# EUROPEAN PATENT APPLICATION

(11) **EP 3 763 290 A1**
(43) Date of publication of application: **13.01.2021**
(21) Application number: 19315068.7
(22) Date of filing: 12.07.2019
(51) Int. Cl.: A61B 5/08, A61B 5/00

(54) **SLEEP SENSING AND MONITORING DEVICE**

(71) Applicant: Withings, 92130 Issy-les-Moulineaux (FR)
(72) Inventor: Aimon, Nicolas, 44100 Nantes (FR); Bedetti, Thomas, 92130 Issy LES Moulineaux (FR); Edouard, Paul, 75015 Paris (FR); Bartet, Pierre, 91570 Bievres (FR); Sanath Koumar Radja, Varoun, 92130 Issy LES Moulineaux (FR); Juan, Paul-Edouard, 75019 Paris (FR)
(74) Representative: Plasseraud IP

(57) **Abstract**

The disclosure is directed to a sensing device, configured to be installed in a bedding, for monitoring a user's sleep, the device comprising:
- a sensing part, for acquiring/determining a value representative of an a force or pressure and/or a value representative of a variation of a force or pressure,
- a housing comprising at least a pressure transducer and an electronic processing unit,
- a microphone connected to the electronic processing unit,
- wherein the electronic processing unit is configured to process first and second electrical signals delivered respectively by the microphone and the pressure converter, wherein the electronic processing unit is either configured to deduce locally at least a breathing disturbance therefrom or configured to send data representative of the first and second electrical signals to a remote device.

## Description

### FIELD OF THE DISCLOSURE

The present disclosure relates to devices and systems for sensing and monitoring sleep of a user.

Monitoring sleep of an individual user provides useful information for such user. Notably when the user may undergo some sleep disorder or trouble or poor quality sleep, sometimes without knowing it, in-depth monitoring can reveal some light or severe sleep disorder, and can help improve the user's sleep quality.

### BACKGROUND OF THE DISCLOSURE

There has been proposed various detection devices designed to allow sleep monitoring, such detection devices are usually placed in the bedding. More specifically, this type of detection device can be installed above a mattress and under a mattress pad or cover; it can also be installed under a mattress topper or below the main mattress or under any other layer; the person to be monitored is intended to be lying down on top of the detection device.

Such detection devices are configured to capture the user's movements and allow determining by ballistography analysis, from pressure changes induced by the user's movements, the heart rate and respiratory rate of the individual, and also data reflecting amplitude of breathing.

EP2873368 illustrates one example of such a device.

However, it is not possible with such detection devices to reliably detect certain sleep problematic phases like sleep apnea and sleep hypopnea.

### SUMMARY OF THE DISCLOSURE

According to one aspect of the present invention, it is disclosed a sensing device, configured to be installed in a bedding, for monitoring a user's sleep, the device comprising:
- a sensing part, for acquiring/determining a value representative of a force or pressure and/or a value representative of a variation of a force or pressure,
- a housing comprising at least a force or pressure converter and an electronic processing unit,
- a microphone connected to the electronic processing unit,
- wherein the electronic processing unit is configured to process first and second electrical signals delivered respectively by the microphone and the force or pressure converter, wherein the electronic processing unit is either configured to deduce locally at least a breathing disturbance therefrom or configured to send data representative of the first and second electrical signals to a remote device.

Thanks to this arrangement, the combined analysis of sounds and movements (either large, medium or tiny movements) can be used to readily and accurately detect/identify a breathing disturbance.

Under the term "*force or pressure*", it shall be equivalently understood an effort exerted by the user body. Similarly, under the term *"variation of a force or pressure",* it shall be understood an effort difference.

Under the term *"breathing disturbance",* it shall be understood a sleep apnea sequence or a hypopnea sequence.

In use, the effort is exerted by the user's body (UB) lying on the sensing part.

Under the term "*pressure converter*", it shall be understood any device able to convert an effort (mechanical force) into an electrical value, e.g. a voltage, to be processed by a control unit. Such pressure converter may be involved whatever the type of sensing part, either pneumatic, or piezoelectric, or otherwise.

It shall be understood that the sensing part can be either piezoelectric (one or more piezoelectric elements) or can be pneumatic (i.e. an air bladder for sensing air pressure therein).

In particular, the inventors have found out that the microphone, even though it is arranged within the housing which is intended to be placed in the bedding, give nonetheless very useful outputs. Preferably, the housing is a unique housing.

It shall be noted that the apnea can be a central apnea phenomenon or an obstructive apnea phenomenon, or mixed. In the central apnea phenomenon, the nervous system does not command the breathing cycle. In the obstructive apnea phenomenon, the nervous system does command the breathing cycle, but the mouth and/or larynx of the user prevent air passage, or hinder severely such air passage.

According to the American Academy of Sleep Medicine (2017), see Kapur VK, Auckley DH, Chowdhuri S, Kuhlmann DC, Mehra R, Ramar K, Harrod CG, Clinical practice guideline for diagnostic testing for adult obstructive sleep apnea: an American Academy of Sleep Medicine clinical practice guideline. J Clin Sleep Med. 2017;13(3):479-504, hypopnea is defined as a reduction of 30% or more of the respiratory signal during 10s or more, coupled with a reduction of at least 3% of the oxygen saturation and/or micro-awakening events. Apnea is defined by a reduction of 90% or more of the respiratory signal during 10s or more. under the term *"hypopnea",* we understand periods of reduction of the breathing flux during a sleep, by contrast with periods of complete obstruction of the breathing flux that is rather qualified as *"apnea".*

It shall be noted that the promoted sensing arrangement is a fortiori capable for snoring detection and normal breathing detection.

In various embodiments of the invention, one may possibly have recourse in addition to one and/or other of the following arrangements, taken alone or in combination.

According to one aspect, the microphone (1) is housed into the housing (6). Said otherwise the microphone is integrated into/within the housing. Thereby, the mechatronics integration is optimized and the solution is cost effective. Further, the microphone is protected from mechanical constraints.

According to one aspect, the microphone (1) can be arranged in any part of the sensing device, for example the microphone (1) can be arranged either next to a connection cable (7) or integrated into a connector to provide power supply.

According to one aspect, the electronic processing unit may be configured to calculate a apnea/hypopnea index (AHI) over a night. The apnea hypopnea index (AHI) is the mean frequency of apnea and hypopnea events over a night expressed in events per hour.

Advantageously, this index gives a complete picture of the user behavior and type of disorder regarding apnea/hypopnea. This index represents an average of breathing disturbance occurrences over a complete period of sleep. This index proves to be a relevant index to qualify the user behavior and type of disorder he/she may have.

According to one aspect, the housing may have a thickness which is less than 20mm, preferably less than 15 mm. The device can therefore be installed in a bed/bedding without exhibiting a large protrusion. The device can be undetectable and discreet. The user can roll over the housing without notable hindrance.

According to one aspect, the sensing part is formed as a sensory band, having a thickness which is less than the thickness of the housing. The sensing surface, which is the surface of the sensory band, can be large enough to accommodate various user's positions. The sensory band can be undetectable and discreet within the bedding.

According to one aspect, there may be provided a microphone hole arranged in the housing, and the sensitive portion of the microphone is arranged opposite the microphone hole. Thereby, the microphone faces outwardly and can thus efficiently sense the external sounds without barrier effect from the housing. Even though the housing and microphone are covered by a cover lining made in fabric, it permits sounds to pass through with little attenuation (no substantive attenuation from the cover lining).

According to one aspect, the microphone is fixed on a flexible printed circuit (FPC) and the FPC is glued on an internal face of the housing, next to the microphone hole. This variant is a simple solution to arrange the microphone at the housing wall, independently of the position of the mother board (PCB).

According to one aspect, the electronic processing unit may be configured to determine pressure changes from the second electrical signals, for example by derivative processing, digital or analog. Starting from pressure changes, even with low voltage signals, we may use amplification stage so to work on easy to handle signals. For pneumatic solution, working on pressure changes renders the downstream process independent of absolute pressure in the bladder.

According to one aspect, the sensing part comprises one or more piezoelectric elements and the pressure converter converts piezoelectric voltage into a converted voltage that can be inputted in the electronic processing unit. This forms a simple all electronic solution, no pump is required.

According to an alternative solution, the sensing part is pneumatic, and the pressure converter is formed as a pressure transducer that converts pressure values into a converted voltage that can be inputted in the electronic processing unit. Thereby, only one sensor can reliably sense the movements, whatever the position of the user.

According to an aspect regarding the pneumatic configuration, the sensing part comprises a pneumatic chamber, which may otherwise be called an air bladder, and the housing comprises a pump and a motor, and preferably the housing further comprises a purge valve. The pneumatic chamber can be inflated to an optimal pressure for the sensing function. The pneumatic chamber can be deflated before rolling the device and stowing. The pump is driven by the motor, controlled by the electronic processing unit.

According to an aspect regarding the pneumatic configuration, the sensing device may further comprise one or more pipes for fluidly coupling together at least the pneumatic chamber, the pump and the pressure transducer. Advantageously the piping is enclosed and protected within the housing.

According to one aspect, the sensing device exhibits a rectangular overall shape (LX, LY) with LX comprised between 50mm and 800mm, and LY comprised between 10mm and 400mm. The sensitive area can be large enough to accommodate various user's positions. It does not exceed popular bedding horizontal sizes and therefore can be aesthetically integrated into a bed.

According to one aspect, the sensing device may further comprise a rechargeable electric battery. Thereby, in operation, the device does not need to have a wire connection. Recharge can be carried out through a wire or through a magnetic flux coupling.

According to one aspect, a data set regarding sleep can be transmitted by the sensing device to a remote entity by a wireless coupler, Bluetooth, Wifi or similar.

According to one aspect, the sensing device may further comprise a connection cable with one or more wire and a connector. Such connector may be a USB connector or any type of plug to provide electrical supply to the sensing device.

The disclosure is also directed at a system comprising a sensing device as described above and a smartphone / remote device comprising an application to display results and user's history.

The disclosure is also directed at a method configured to be carried out by a system comprising a sensing device as described hereinabove, the sensing device comprising at least a sensing part, a microphone, a housing comprising at least a force or pressure transducer and an electronic processing unit, the method comprising:
- installing the sensing device in a bedding, for monitoring a user's sleep,
- acquiring/determining, by the sensing part, a value representative of a force or pressure and/or a value representative of a variation of a force or pressure,
- processing, by the electronic processing unit, a first and second electrical signals delivered respectively by the microphone and the pressure converter of the sensing device,
- either deducing locally, by the electronic processing unit at least a breathing disturbance therefrom or sending data representative of the first and second electrical signals to a remote device.

### BRIEF DESCRIPTION OF THE DRAWINGS

Other features and advantages of the invention appear from the following detailed description of two of its embodiments, given by way of non-limiting example, and with reference to the accompanying drawings, in which:
- Figure 1 illustrates a diagrammatical perspective view of a sensing device according to a first embodiment,
- Figure 2 shows an elevation sectional view of the device of figure 1,
- Figure 3 shows a transverse sectional view of the device of figure 1,
- Figure 4 illustrates a block diagram of the device of figure 1,
- Figure 5 illustrates a partial diagrammatical exploded view of the device of figure 1,
- Figure 6 illustrates a diagrammatical perspective view of the housing,
- Figure 7 shows an elevation sectional view of the housing, with notably the microphone arrangement,
- Figure 8 illustrates a diagrammatical graph of apnea basic index over the time calculated by means of the sensing device of figures 1 to 7,
- Figure 9 illustrates a diagrammatical top view of a sensing device according to a second embodiment,
- Figure 10 shows a side sectional view of the device of figure 9,
- Figures 11 and 12 illustrate diagrammatically two variants of a transverse sectional view of the device of figure 9.
- Figure 13 illustrates two variants for the sensing device of figure 1, wherein the microphone is disposed in variant locations on the sensing device.

### DETAILED DESCRIPTION OF THE DISCLOSURE

In the figures, the same references denote identical or similar elements. It should be noted that, for clarity purposes, some element(s) may not be represented at scale.

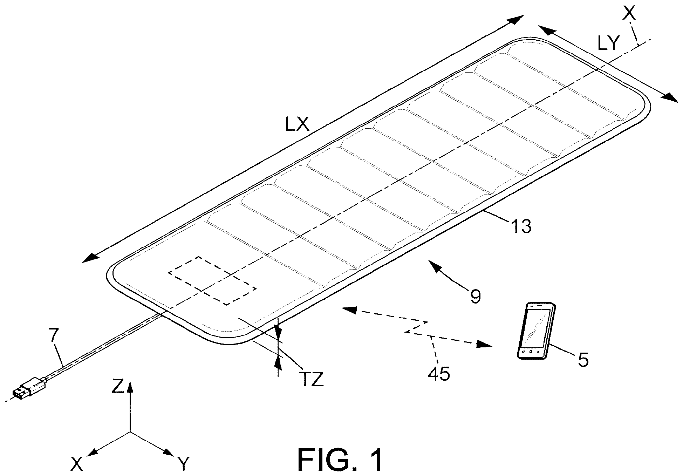

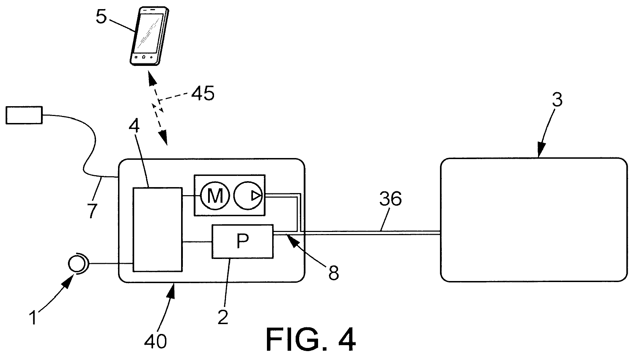

Figures 1 to 4 show a sensing device according to a first embodiment.

The sensing device 9 comprises a band 13 of fabric enclosing means for detecting apnea or hypopnea of a user during a sleep. The sensing device 9 may be stowable, either rolled or folded in three parts such that it is compact when not used. In the folded configuration, the sensing device 9 has a length of about 22cm, a width of about 18cm and a thickness of about 5cm. In the rolled configuration, the sensing device 9 has a length of about 13cm and a diameter of about 6cm.

The band 13 of fabric is intended to be installed under a mattress. When installed under the mattress, the user sleeping over the mattress would not be disturbed by the sensing device 9 presence. Indeed, the total thickness (TZ) of the band 13 is low enough to be undetectable under the mattress. For instance, the total thickness is about 20mm or less. In use, it can be [10-20mm]. In order to detect the apnea or hypopnea of the user during a sleep, the band 13 encloses a pneumatic chamber 3 acting as a sensing part.

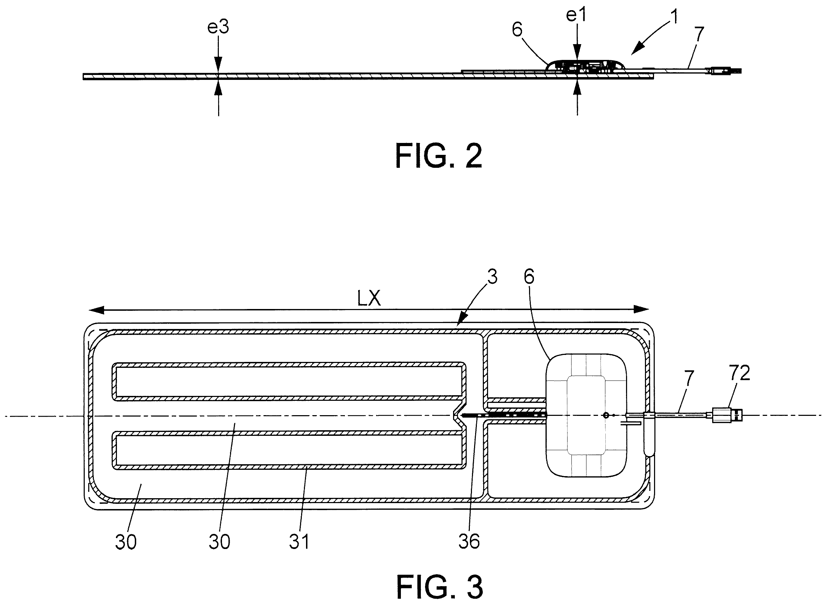

The pneumatic chamber 3 is represented more in detail on Figures 2 and 3. As one can see, the shape of the pneumatic chamber 3 is sensibly the same as the shape of the band 13, i.e. rectangular, and has an upper layer and a lower layer that vertically delimit the pneumatic chamber 3. The width LY of the rectangular shape is about 200mm, and the length LX is about 600mm. The pneumatic chamber 3 comprises some fluidly interconnected chambers portions 30 which are separated to each other by means of braces 31 joining two opposite faces of the chamber 3. For instance, the braces 31 are formed by heat melting the upper and lower layers of the pneumatic chamber 3. The chamber portions 30 therefore form some comfortable inflated tubes.

Some foam is included in the band 13 of fabric in order to make it further comfortable. Advantageously, the band 13 of fabric is removable and made into a material of fabric that is washable. For instance, the material is made of polyester and has a coating in thermoplastic polyurethane (TPU).

The chamber 3 has a total thickness e3 about 5mm.

In order to get, analyze and transmit signals representative to apnea or hypopnea, the band 13 encloses a housing 6 comprising a pressure transducer 2. The pressure transducer 2 is connected with the chamber 3 via an air tube 8. The air tube 36 enables air connection even if the chamber 3 is pressed near the housing 6. Hence, the pressure can be transmitted through the air tube 36.

Alternatively, the air variation can pass through a channel coupling the air chamber with the pressure transducer, said channel having an anti-collapse rod therein.

The housing 6 further comprises electronic means. The electronic means namely comprise an electronic processing unit 4. The electronic means are further adapted for communicating with a smartphone 5, as represented by the dotted-line arrow 45. The electronic means are also connected to a connection cable 7 comprising a wire and a USB connector 72 in order to be able to electrically feed the sensing device 9.

Further, the housing 6 comprises a microphone 1, also referred to as an audio sensor. The microphone is configured to acquire sounds, and in particular breathing or snoring sounds.

Both the microphone 1 and the pressure transducer 2 are electronically connected to the electronic processing unit 4.

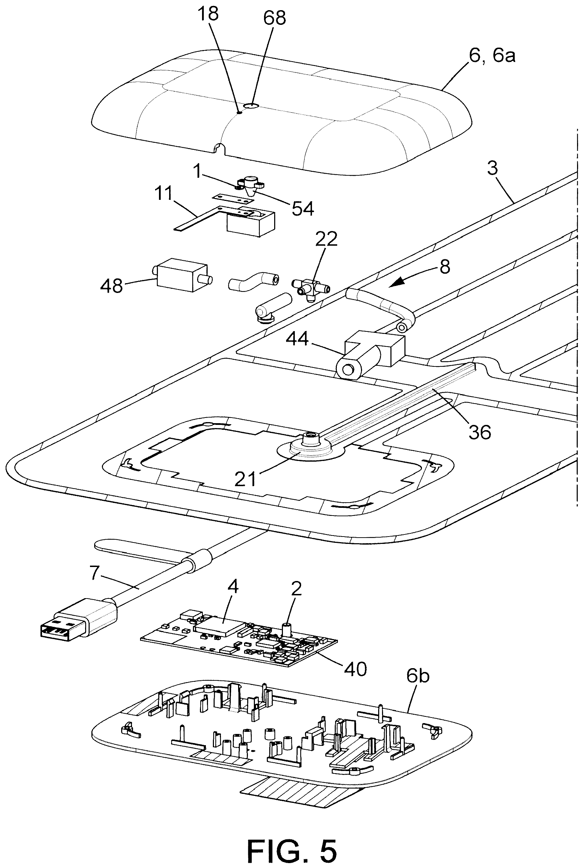

Figures 5 to 7 represent more in detail the housing 6. The housing 6 is made of plastic. The thickness e1 of the housing 6 is less than 15 mm.

The housing 6 comprises an upper shell 6a and a lower shell 6b that cooperate together in order to form an unfoldable rigid protection for the electronic means. For instance, the upper shell 6a and lower shell 6b are assembled by snap-fit assembly method or by ultrasonic weld assembly.

Inside the rigid protection, the housing 6 also comprises a Printed Circuit Board (PCB) 40. The pressure transducer 2 and the electronic processing unit 4 are disposed and connected to the PCB 40. The microphone is disposed higher than the plan surface of the PCB 40, on a support near the internal surface of the upper shell 6a. The microphone 1 is connected to the electronic processing unit 4 by means of a foldable flexible circuit board (FPC) 11. The upper shell 6a comprises an hole 18 formed on the supper surface in order to face the microphone 1 and enable the microphone 1 to get the sounds from the outside of the housing 6.

Therefore, the housing 6 forms a complete and secured enclosure that houses the microphone 1, the pressure converter 2 and the electronic processing unit 4.

Advantageously, a light emitting device (LED) 54 can also be included in the housing 6, and disposed on the PCB 40, in order to produce a lighting signal when the sensing device 9 is in use. Advantageously, the upper shell 6a further comprises a lighting hole 68 facing the LED 54 in order to diffuse the light through the plastic of the housing 6.

The upper shell 6a may further comprise a reset hole for accessing a switch electronically connected to the PCB 40.

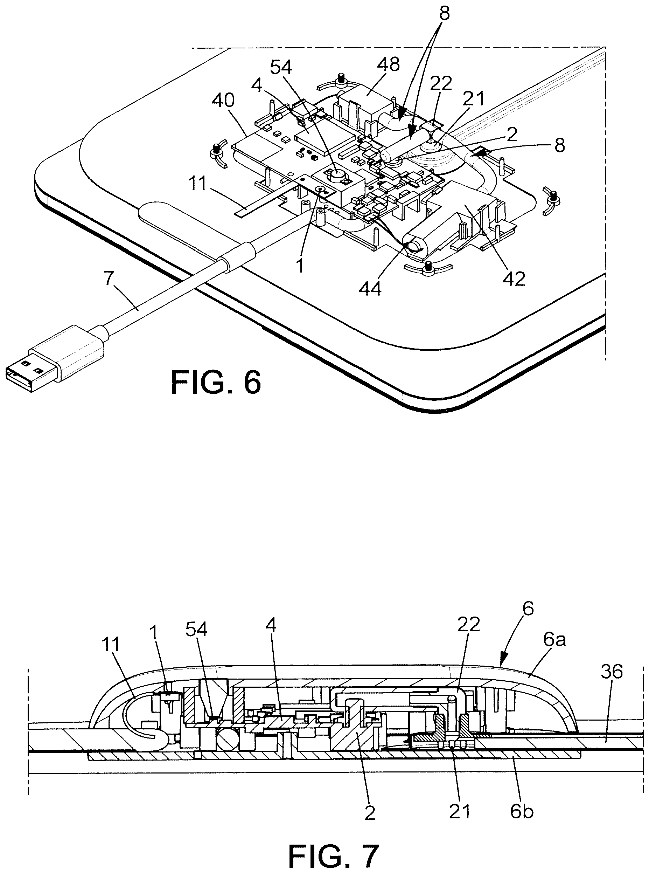

One can see on figure 6 that the chamber 3 is connected to an input of a pneumatic connector 21 through the air tube 36. The pneumatic connector 21 has a "T" shape three-channel output 22, each of the channels being fluidly connected to a pipe 8.

One of the pipes 8 is fluidly connected to a purge valve 48, such that the chamber 3 forms an air bladder. One of the pipes 8 is fluidly connected to a pump 42 able to inflate the chamber 3. A motor 44 is provided for rotating the pump 42. One of the pipes 8 is fluidly connected to the pressure transducer 2 in order to convert detected pressure into voltage.

All the pipes 8 and fluidly connected pneumatic connector 21, purge valve 48, pump 42 and pressure transducer 2 are also enclosed and protected within the same unique housing 6.

The chamber 3 may be inflated prior to be used during the sleep time, for instance prior the night, by means of the pump 42 and the motor 44.

Therefore, when lying on the mattress, the weight and movements of the user act on the pneumatic, which results on pressure variations. The microphone gets the sounds of the surroundings, namely breathes or snoring, and converts them into a first voltage. The pressure transducer 2 simultaneously gets the pressure variations and converts it into a second voltage.

The electronic processing unit 4 is configured for analyzing both the first and second voltage and to calculate a apnea/hypopnea index (AHI) over a night, for the user.

Alternatively, the electronic processing unit 4 is configured for sending the first and second voltage to the smartphone 5 which is configured either to calculate the AHI or to send the first and second voltage to a remote server which is configured to perform said calculations.

For instance, the calculations are performed by means of a trained artificial intelligence (AI). The AI is trained by feeding an algorithm with data sets comprising tagged records of sounds and of pressure variations. The tags enable to classify the records. For instance, a set comprising a record of sounds and a record of pressure variation of a user during a whole night may be associated to the following tags:
- weight of the user,
- age of the user,
- type of apnea or AHI measured by a polysomnograph and read by a trained medical professional.

More specifically, for the AI training purpose, records of sounds and pressure variation are performed by a sensing device according to the invention, on a plurality of study patients. A doctor identifies, for each study patient, a type of breathing disturbance thanks to medical monitoring of various physiological signals, called polysomnography (PSG). The most importantly signals of the PSG used for the diagnosis of sleep apnea are the air flux in the breathing of the study patient, snoring, movements of the thorax and abdomen, and the O2 saturation in the blood of the study patient. The different types of breathing disturbances identified by the doctor may be hypopnea, apnea of mix of hypopnea and apnea, and may be numbered over a night by the AHI. The different types of apnea may be obstructive, central, or mixed. This gives a reference database, which can be improved/ increased over time, and patient monitoring.

Therefore the doctor may tag the records of sounds and of pressure variation in association with the profile, i.e. for instance the age, gender and weight, of each study patient.

In use, the trained AI is able to estimate, or "predict", an AHI from the pressure and sound recordings of the device of the present invention.

The user may install a dedicated application on his smartphone 5. The dedicated application may display graphs of variation of the frequency of apnea/hypopnea episodes in the course of the night. The application may also display the AHI, which represents the average number of apnea/hypopnea episodes per hour in the night.

The application may also display a graph of an apnea basic index 12 over the time on the screen of the smartphone 5.

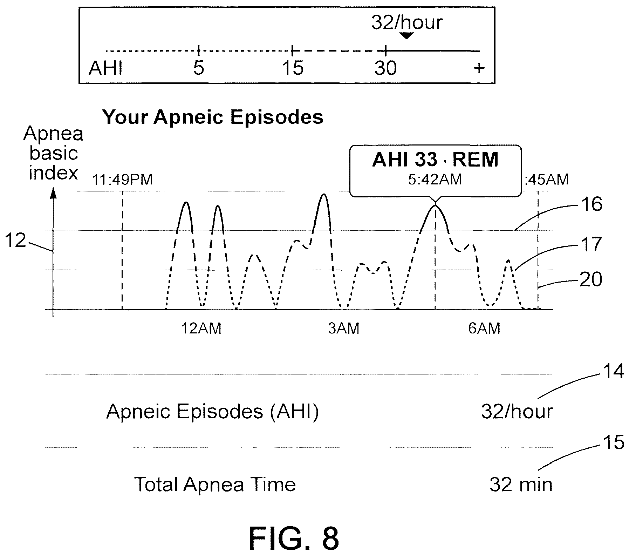

The graphical user interface of the application is pictured on figure 8.

The apnea basic index 12 represents the number 14 of likely apneic episodes during an elementary time division. As represented, for a time division equal to an hour, the number 14 in the displayed example is 32 per hour.

The graph of apnea basic index 12 is divided in three categories of number 14 of episodes per hour: low number 20, medium number 17 and high number 16, corresponding to the medical classification of gravity of the apnea, respectively, light apnea (0 to 15 episodes), moderate apnea (15 to 30 episodes) and severe apnea (over 30 episodes). A "total apnea time" represents a cumulated time lapse 15 over the sleep during which the number 14 is high, i.e. is in the high part 16 on the graph. When the total apnea time is high, for instance is over 30min, then the probability that the user suffers from apnea - in other words, that the user could be diagnosed as an apneic person - is high

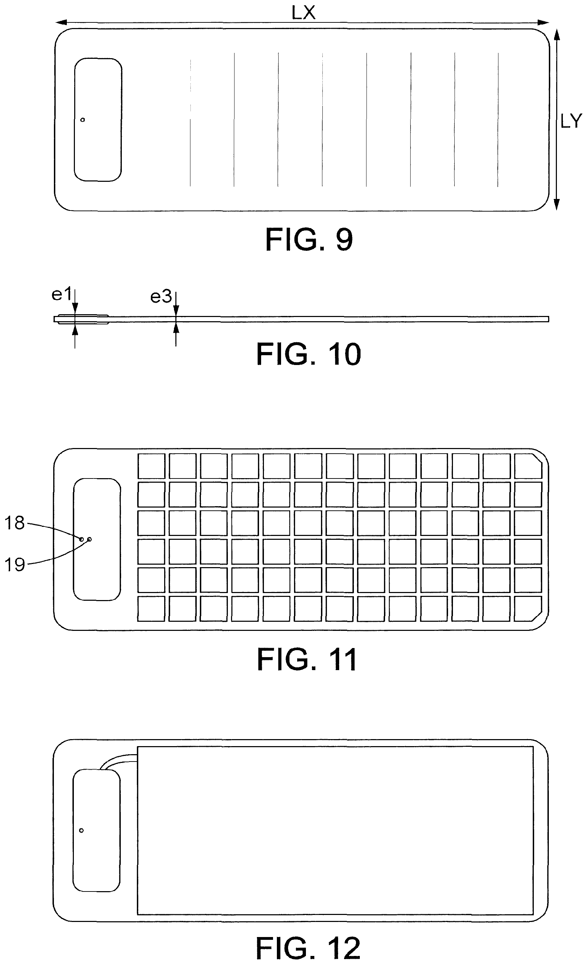

The figures 9 and 10 show an alternative embodiment to the embodiment described with reference to the figures 1-4. The overall difference is that the sensing part is not made from a pneumatic chamber 3 but made from a series of parallel lined piezoelectric elements. One can see on the figures that the parallel lines are transversally extending. In another embodiment (not represented on the figures), one can design parallel lines longitudinally extending as well.

The pressure converter 2 converts the piezoelectric voltage into a converted voltage that can be inputted in the electronic processing unit 4.

The figure 11 shows an alternative embodiment to the embodiment described with reference to the figures 1-4. The only difference is that the sensing part is not made from a pneumatic chamber 3 but made from a matrix of piezoelectric elements. The pressure converter 2 in this embodiment converts the piezoelectric voltage into a converted voltage that can be inputted in the electronic processing unit 4.

The figure 12 shows a further alternative embodiment. The only difference with the embodiment described with reference to the figure 11 is that the sensing part is made from a unique large piezoelectric element.

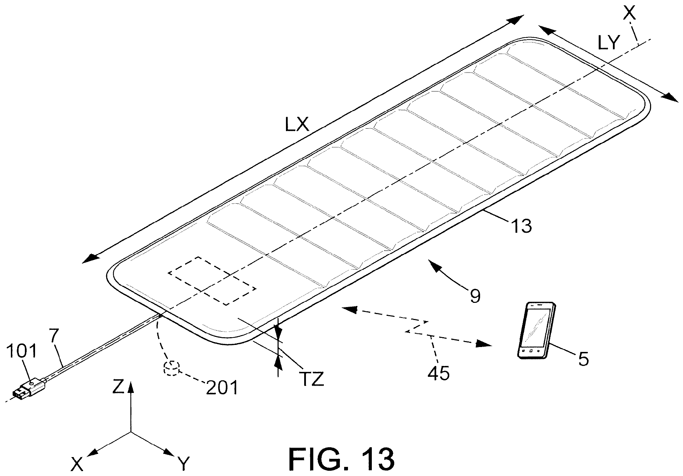

One can see on the figure 13 two alternative dispositions of the microphone 1, by comparison with the above description.

For instance, instead of being enclosed in the housing 6, a microphone 201 may be either disposed on the connection cable 7.

According to another arrangement, there is provided a microphone 101 disposed on the USB connector 72. The microphone 101 may have the same functional behaviors as per above embodiments.

## Claims

1. A sensing device (9), configured to be installed in a bedding, for monitoring a user's sleep, the device comprising:
- a sensing part (3), for acquiring/determining a value representative of a force or pressure and/or a value representative of a variation of a force or pressure,
- a housing (6) comprising at least a force or pressure converter (2) and an electronic processing unit (4),
- a microphone (1) connected to the electronic processing unit (4),
- wherein the electronic processing unit (4) is configured to process first and second electrical signals delivered respectively by the microphone and the force or pressure converter, wherein the electronic processing unit (4) is either configured to deduce locally at least a breathing disturbance therefrom or configured to send data representative of the first and second electrical signals (51,52) to a remote device.

2. The sensing device according to claim 1, wherein the microphone (1) is housed into the housing (6).

3. The sensing device according to claim 1 or 2, wherein the electronic processing unit (4) is configured to calculate a apnea/hypopnea index (AHI) over a night.

4. The sensing device according to any of the claims 1 to 3, wherein the housing has a thickness (e1) which is less than 20mm, preferably less than 15 mm.

5. The sensing device according to claim 4, wherein the sensing part is formed as a sensory band (3), having a thickness (**e3**) which is less than the thickness (e1) of the housing.

6. The sensing device according to claim2, wherein there is provided a microphone hole (18) arranged in the housing, and the sensitive portion of the microphone is arranged opposite the microphone hole.

7. The sensing device according to any of the claims 1 to 6, wherein the sensing part comprises one or more piezoelectric elements and the pressure converter (2) converts piezoelectric voltage into a converted voltage that can be inputted in the electronic processing unit (4).

8. The sensing device according to any of the claims 1 to 6, wherein the sensing part is pneumatic, and the pressure converter is formed as a pressure transducer that converts pressure values into a converted voltage that can be inputted in the electronic processing unit.

9. The sensing device according to claim 8, wherein the sensing part comprises a pneumatic chamber (3) and the housing (6) comprises a pump (42) and a motor (44), and preferably the housing further comprises a purge valve (48).

10. The sensing device according to claim 9, further comprising pipes (8) for fluidly coupling together at least the pneumatic chamber, the pump and the pressure transducer.

11. The sensing device according to any of the claims 1 to 10, wherein said device exhibits a rectangular overall shape (LX, LY) with LX comprised between 50mm and 800mm, and LY comprised between 10mm and 400mm.

12. The sensing device according to any of the claims 1 to 11, further comprising a rechargeable electric battery.

13. The sensing device according to any of the claims 1 to 12, further comprising a connection cable (7) with one or more wire and a connector.

14. A system comprising a sensing device (9) according to any of the claim 1 to 13, and a smartphone / remote device (5) comprising an application to display results and user's history.

15. A method configured to be carried out by a system comprising a sensing device (9) according to claim 1 to 14, the sensing device comprising at least a sensing part (3), a microphone (1), a housing (6) comprising at least a force or pressure transducer (2) and an electronic processing unit (4), the method comprising:
- installing the sensing device in a bedding, for monitoring a user's sleep,
- acquiring/determining, by the sensing part (3), a value representative of a force or pressure and/or a value representative of a variation of a force or pressure,
- processing, by the electronic processing unit (4), a first and second electrical signals (51, 52) delivered respectively by the microphone and the pressure converter of the sensing device,
- either deducing locally, by the electronic processing unit (4) at least a breathing disturbance therefrom or sending data representative of the first and second electrical signals (51,52) to a remote device.
